# FASCICULE DE BREVET EUROPEEN

(11) **EP 4 114 324 B1**
(45) Date de publication et mention de la délivrance du brevet: **14.02.2024**
(21) Numéro de dépôt: 21713727.2
(22) Date de dépôt: 26.02.2021
(51) Int. Cl.: A61F 2/78, A61F 5/01, A61F 2/80, A61F 2/50

(54) **APPAREILLAGE MEDICAL FLEXIBLE ET PROCEDE DE FABRICATION D'UN TEL APPAREILLAGE**
FLEXIBLE MEDIZINISCHE VORRICHTUNG UND VERFAHREN ZUR HERSTELLUNG EINER SOLCHEN VORRICHTUNG
FLEXIBLE MEDICAL DEVICE AND METHOD OF MANUFACTURING SUCH A DEVICE

(30) Priorité: 06.03.2020 FR 2002261
(43) Date de publication de la demande: 11.01.2023
(73) Titulaire: Paillet, Stéphane, 26230 Valaurie (FR)
(72) Inventeur: Paillet, Stéphane, 26230 Valaurie (FR)
(74) Mandataire: IP Trust
(86) Numéro de dépôt international: PCT/FR2021/050331
(87) Numéro de publication internationale: WO 2021/176165

(56) Documents cités:
- EP-A1- 2 856 980
- FR-A1- 2 539 616
- FR-A1- 2 828 093
- US-A1- 2019 046 338

## Description

### DOMAINE DE L'INVENTION

La présente invention concerne un appareillage médical flexible destiné à être disposé sur une partie du corps d'un patient ainsi que son procédé de fabrication. Elle concerne plus particulièrement le domaine des appareillages prothétiques ou orthopédiques.

### ARRIERE PLAN TECHNOLOGIQUE DE L'INVENTION

Une prothèse est un appareillage médical qui a pour but de remplacer un membre manquant ou une partie de ce membre. Elle est généralement constituée d'une emboîture à laquelle est fixé le membre synthétique de remplacement. L'emboîture est destinée à recevoir l'extrémité du membre amputé et permet son raccordement au membre synthétique. L'emboîture peut être fabriquée à façon afin de s'adapter à la forme et la taille du membre amputé.

Comme le rappel le document EP2856980, les emboîtures sont généralement réalisées en résine ou en plastique rigide ou semirigide, et comportent une structure externe à base de carbone. Or l'emboîture doit être confortable, facile à installer et avoir une bonne tenue sur le membre amputé, afin notamment de ne pas la perdre lors d'un déplacement.

Pour résoudre cela, il est connu de l'état de la technique d'utiliser des emboîtures comprenant un manchon, le plus souvent en silicone, placé à l'intérieure de l'emboîture. Le silicone est un matériau souple, déformable et confortable. Le manchon facilite la mise en place du membre amputé dans l'emboîture. Il a également une fonction de confort, le manchon assurant le contact et l'amortissement du membre amputé dans l'emboîture, en réduisant les douleurs éventuelles.

Néanmoins ce type d'emboîture présente plusieurs inconvénients. Il ne permet pas de prendre en compte les variations de volume du moignon. L'emboîture peut être inconfortable en position assise à cause de sa rigidité. La mise en place du manchon quotidiennement à chaque utilisation de la prothèse peut également être une contrainte à cause de son éventuelle complexité.

On connaît une solution à ces inconvénients des documents EP2856980 et FR2539616 qui présentent chacun une emboîture comprenant des zones souples et des zones rigides formant ainsi une emboîture flexible capable de se déformer et de s'adapter à différentes sollicitations. Plus particulièrement, l'emboîture de ces documents comprend une armature rigide prise en sandwich par deux couches de matériau souple en silicone. Toutefois, ce type d'emboîture présente l'inconvénient d'être lourd en raison de la quantité importante de matériau souple employée, le silicone étant un matériau très dense.

### OBJET DE L'INVENTION

Un but de l'invention est de proposer un appareillage médical remédiant au moins en partie à cet inconvénient. Plus particulièrement, l'invention porte sur un appareillage médical flexible et léger ainsi que sur son procédé de fabrication. L'appareillage peut être une prothèse pour remplacer un membre manquant d'un patient ou une partie de ce membre. Il peut s'agir également d'une orthèse pour suppléer, de manière temporaire ou définitive, une carence ou une déformation ostéoarticulaire ou musculaire du patient.

### BREVE DESCRIPTION DE L'INVENTION

En vue de la réalisation de ce but, l'objet de l'invention propose un appareillage médical flexible destiné à être disposé sur une partie du corps d'un patient et en épouser la forme, l'appareillage médical flexible comprenant une structure rigide présentant un contour ainsi qu'au moins une couche déformable.

Selon l'invention, un tissu de liaison est engagé dans au moins une portion périphérique de la structure rigide s'étendant le long de son contour, le reste du tissu de liaison formant une portion exposée pour recevoir la couche déformable. La structure rigide et la couche déformable sont assemblées bord à bord, la couche déformable complétant la structure rigide pour former l'appareillage médical.

Le tissu de liaison, engagé dans la structure rigide et retenant le matériau souple de la couche déformable, permet de placer bord à bord la partie souple et la partie rigide de l'appareillage. Il n'est donc pas nécessaire de retenir en sandwich la structure rigide par deux couches de matériau souple, ce qui réduit la quantité de matériau souple et le poids de l'ensemble.

Selon d'autres caractéristiques avantageuses et non limitatives de l'invention, prises seules ou selon toute combinaison techniquement réalisable :
- le matériau de la structure rigide est un matériau composite à base de carbone ;
- le matériau du tissu de liaison est de la fibre de verre ;
- le matériau de la couche déformable est un matériau élastomère tel qu'un silicone ;
- la couche déformable et la structure rigide ne se recouvrent pas ;
- L'appareillage médical comprend un film de revêtement interne en matériau déformable, le film de revêtement interne recouvrant au moins en partie la surface interne de la structure rigide et la couche déformable ;
- L'appareillage médical comprend un film de revêtement externe en matériau déformable, le film de revêtement externe recouvrant au moins en partie la surface externe de la structure rigide et la couche déformable ;
- la structure rigide comprend au moins deux mâts s'étendant sensiblement axialement depuis une base vers une extrémité.

Selon un autre aspect, l'invention propose un procédé de fabrication d'un appareillage médical flexible destiné à être disposé sur une partie du corps d'un patient et en épouser la forme, le procédé comprenant les étapes suivantes :
- fournir un positif de la partie du corps destiné à recevoir l'appareillage médical ;
- former une structure rigide, munie d'un insert, sur une portion seulement du positif pour préserver une portion du positif, dite portion libre ;
- former une couche déformable sur la portion libre du positif, la formation de la couche déformable comprenant les étapes suivantes :
   o engager une portion d'un tissu de liaison dans l'insert de la structure rigide, le reste du tissu de liaison formant une portion exposée recouvrant au moins partiellement la portion libre du positif ;
   o former la couche déformable sur la portion exposée du tissu de liaison et bord à bord de la structure rigide ;
   o appliquer un traitement thermique pour durcir la couche déformable.

Selon d'autres caractéristiques avantageuses et non limitatives de l'invention, prises seules ou selon toute combinaison techniquement réalisable :
- la formation de la structure rigide comprend les étapes suivantes :
   o former une première couche en un matériau apte à être rigidifié, la première couche présentant un contour ;
   o déposer un film de séparation sur au moins une partie de la première couche pour recouvrir au moins une zone périphérique s'étendant le long du contour de cette première couche ;
   o former une deuxième couche en un matériau apte à être rigidifié en recouvrement de la première couche, de sorte que le film de séparation soit disposé entre la première et la deuxième couche au niveau au moins de la zone périphérique ;
   o appliquer un traitement thermique à l'ensemble formé de la première couche, du film de séparation et de la deuxième couche pour obtenir la structure rigide, le film de séparation prévenant le scellement de la première couche à la deuxième couche au moins dans la zone périphérique pour définir un insert de la structure rigide ;
   o extraire le film de séparation de la structure rigide ;
- la formation de la première couche et de la deuxième couche comprend le dépôt d'une pluralité de couches intermédiaires en matériau apte à être rigidifié ;
- la formation de la structure rigide comprend, une étape de finition, l'étape de finition comprenant :
   o le retrait du positif;
   o le détourage de la structure rigide pour conformer le contour de la deuxième couche à celui de la première couche;
   o le ponçage des bords de la structure rigide afin que les bords présentent une surface compatible avec un collage bord à bord ;
   o la remise en place du positif ;
- le procédé de fabrication d'un appareillage médical comprend une étape préliminaire, avant la formation de la structure rigide, de formation d'un film de revêtement interne en matériau déformable, cette étape préliminaire comprenant :
   o le dépôt du film de revêtement interne sur toute la surface du positif ;
   o un traitement thermique pour durcir le film de revêtement interne ;
- l'étape préliminaire comprend, à la suite de son traitement thermique, le dépôt d'un film d'isolation sur le film de revêtement interne pour l'isoler du reste de l'appareillage médical lors de la formation de la structure rigide, le film d'isolation étant retiré à l'issue de la formation de la structure rigide ;
- le procédé de fabrication d'un appareillage médical comprend une étape supplémentaire de dépôt d'un film de revêtement externe en matériau déformable pour recouvrir la surface externe de la structure rigide ainsi que celle de la couche déformable ;
- l'étape supplémentaire de formation d'un film de revêtement externe est réalisée entre le dépôt de la couche déformable et le traitement thermique de l'étape de formation de la couche déformable, le traitement thermique de l'étape de formation de la couche déformable durcissant simultanément la couche déformable et le film de revêtement externe.

### BREVE DESCRIPTION DES FIGURES

D'autres caractéristiques et avantages de l'invention ressortiront de la description détaillée de l'invention qui va suivre en référence aux figures annexées sur lesquels :
- La figure 1 représente une vue d'ensemble d'un appareillage médical conforme à l'invention ;
- La figure 2 représente un positif de la partie du corps d'un patient destinée à recevoir un appareillage médical conforme à l'invention ;
- Les figures 3a à 3d illustrent différentes étapes de la formation d'une structure rigide.
- La figure 4 représente une étape de formation d'une couche déformable.

### DESCRIPTION DETAILLEE DE L'INVENTION

### Présentation générale de l'emboîture

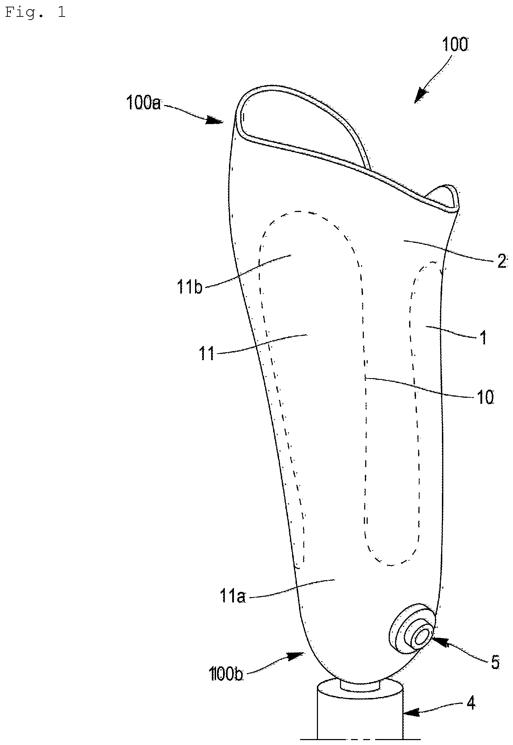

La figure 1 représente une vue d'ensemble d'un appareillage médical 100, ici une emboîture de prothèse, conforme à l'invention.

Dans la suite de la description, on emploiera indifféremment les termes « appareillage médical 100 », « appareillage 100 » ou « emboîture 100 » pour désigner cet appareillage.

Un appareillage médical 100 conforme à l'invention comprend une partie rigide ainsi qu'une partie déformable. Cette hybridation permet de proposer une emboîture 100 suffisamment rigide pour assurer sa tenue sur le moignon tout en ayant suffisamment de flexibilité pour s'adapter à diverses situations, telles que des variations de volume du moignon. La présence de parties flexibles dans l'emboîture 100 permet donc à celle-ci de se déformer pour s'adapter à cette variation de volume, ce qui améliore le confort de l'utilisateur et permet de prévenir les blessures.

L'emboîture 100 est généralement associée à un élément modulaire 4 formant un membre synthétique, ainsi qu'à une valve 5 permettant de chasser l'air de l'intérieur de l'emboîture 100 lors de l'introduction du moignon et de former un vide assurant l'assemblage de l'emboîture au moignon. Mais cette valve 5 ne forme pas un élément essentiel de l'emboîture 100, et elle peut être remplacée par tout autre moyen de suspension du moignon et/ou d'un manchon dans l'emboîture, par exemple un moyen d'accrochage distal. L'emboîture 100 comprend une partie proximale 100a permettant l'insertion du membre amputé ainsi qu'une partie distale 100b. L'élément modulaire 4 ainsi que la valve 5 sont avantageusement situés sur la partie distale 100b de l'emboîture 100.

La partie rigide de l'emboîture est formée d'une structure rigide 1. Cette structure rigide 1 présente un contour 10 et forme l'armature de l'emboîture 100. Elle est formée d'une couche de matériau pouvant présenter une épaisseur typiquement comprise entre 0,5mm et plusieurs millimètres tel que 2 ou 3mm, voir même 5mm. Avantageusement, la structure rigide 1 incorpore la partie distale 100b de l'emboîture 100 pour assurer la solidité de la jonction entre l'emboîture 100 et l'élément modulaire 4. Typiquement, la structure rigide 1 est formée d'un matériau composite comprenant du carbone, par exemple une résine polymère renforcée de fibres de carbone. D'une manière générale, la structure rigide 1 est formée à partir d'une fibre technique (à base de lin, de basalte, d'aramide tel que du kevlar, de verre ou de carbone) et d'une résine polymère ou d'une matière plastique qui renforce cette fibre. La résine ou la matière plastique peut être thermodurcissable ou thermo formable.

La structure rigide 1 peut présenter une grande variété de formes et de contours 10. Pour bien maintenir le membre du patient, elle peut avantageusement comprendre une pluralité de mâts 11, quatre mâts sur l'exemple représenté sur les figures, s'étendant sensiblement axialement depuis une base 11a de la partie distale 100b vers une extrémité 11b du côté de la partie proximale 100a de l'emboîture 100. Les extrémités 11b des différents mâts 11 peuvent indifféremment être reliées ou non entre elles en fonction des besoins et de la rigidité voulue. Lorsque certaines de ces extrémités 11b sont reliées entre elle, elles définissent des ouvertures de la structure rigide 1 délimitant des emplacements pour des zones flexibles. Bien évidemment, la forme représentée sur les figures n'est nullement restrictive, et on pourra donner à la structure rigide 1 toute forme qui convient selon la nature de l'appareillage médical et les besoins du patient.

La partie souple de l'emboîture 100 est formée d'une couche déformable 2, typiquement réalisée dans un matériau élastomère, tel qu'un gel ou une gomme de silicone. Le matériau élastomère peut être un élastomère silicone réticulable à température ambiante, appelé « RTV silicone » en anglais (Room Température Vulcanization), qui est formé par mélange de deux composants, en présence d'un catalyseur, qui réticulent idéalement à une température comprise entre 20 et 25°C. Mais de préférence, le matériau élastomère sera un élastomère silicone réticulable à chaud, appelé « HTV silicone » en anglais (High Température Vulcanization). Le matériau élastomère peut être également un élastomère thermoplastique, un polyuréthane, ou un polyuréthane contenant du fluor, un copolymère.

La couche déformable 2 présente une épaisseur sensiblement équivalente à celle de la structure rigide 1, typiquement entre 2 mm et 5mm, notamment au niveau de la jonction avec cette structure.

La couche déformable 2 complète la structure rigide 1 pour constituer l'appareillage médical 100. La couche déformable 2 peut être formée d'un seul tenant ou comprendre plusieurs parties distinctes, notamment lorsque cette couche emplit des ouvertures formées dans la structure rigide 1. La couche déformable 2 et la structure rigide 1 sont assemblées bord à bord, sans nécessairement se chevaucher, ce qui forme une caractéristique importante de l'appareillage 100. Par « bord à bord », on signifie que la structure rigide 1 et la couche déformable 2 sont jointives et sont en contact l'une avec l'autre par leurs bords.

Le matériau constituant le film déformable 2 n'ayant généralement pas de propriétés adhésives avec le matériau formant la structure rigide 1, et l'étendue des surfaces en contact étant très réduite, l'invention propose d'utiliser un tissu de liaison 3 pour permettre un assemblage robuste, bord à bord, de la couche déformable 2 et de la structure rigide 1.

L'emboîture 100 comprend ainsi un tissu de liaison 3 dont une portion est engagée au moins dans une portion périphérique de la structure rigide 100, cette portion périphérique s'étendant le long de son contour 10. Le reste du tissu de liaison 3, c'est à dire la portion de ce tissu qui n'est pas engagé dans la structure rigide, forme une portion exposée 3'. La portion exposée 3' du tissu de liaison 3 est destiné à recevoir la couche déformable 2, le tissu de liaison permettant ainsi de faire la jonction entre la structure rigide 1 et la couche déformable 2 pour les maintenir assemblées entre elles.

Le tissu de liaison 3 est composé d'un matériau souple et résistant, notamment au cisaillement afin réduire le risque de déchirement à la jonction entre la structure rigide 1 et la couche déformable 2. Il peut s'agir de tout textile technique, tel qu'un textile en fibre de verre, en aramide, en plastique tel que du polyéthylène à haute masse molaire.

Cette configuration d'assemblage bord à bord permet de réduire la quantité de matériau souple formant la couche déformable 2 nécessaire pour former l'emboîture 100 et ainsi réduire son poids, la couche déformable 2 ne recouvrant pas nécessairement la structure rigide 1. Il n'est pas nécessaire, comme c'est le cas dans l'état de la technique, de prendre en sandwich la partie rigide dans la partie souple pour la maintenir assemblée à cette dernière, la surface interne et la surface externe de la partie rigide étant chacune recouverte d'au moins une couche déformable.

Selon une première variante de l'invention, l'emboîture 100 peut avantageusement comprendre un film de revêtement interne en matériau déformable pouvant être de même nature que celui de la couche déformable 2, par exemple en silicone. Le film de revêtement interne recouvre la surface interne de la structure rigide 1 ainsi que celle de la couche déformable 2. Le film de revêtement interne permet d'améliorer le confort de l'utilisateur tout en conservant une emboîture 100 plus légère que celles de l'état de la technique. Le film de revêtement peut présenter une épaisseur réduite, inférieure à 2mm, car il assure une fonction de confort et/ou d'étanchéité, et n'assure aucunement la cohésion de la structure rigide 1 et de la couche déformable 2.

Selon une deuxième variante de l'invention, l'emboîture 100 peut comprendre un film de revêtement externe en matériau déformable, ce film pouvant être de même nature que celui de la couche déformable 2. Le film de revêtement externe recouvre la surface externe de la structure rigide 1 ainsi que celle de la couche déformable 2. Le film de revêtement externe permet d'améliorer l'esthétisme de l'emboîture 100 tout en conservant une emboîture 100 plus légère que l'état de la technique. Tout comme le film de revêtement interne, le film de revêtement externe peut présenter une épaisseur réduite, inférieure à 2mm.

On peut bien entendu envisager de combiner ces deux variantes et de proposer une emboîture comprenant à la fois un film de revêtement interne et externe. Ces films étant peu épais, on préserve le gain de poids vis-à-vis des solutions de l'état de la technique.

### Procédé de fabrication de l'appareillage médical

Le procédé de fabrication de l'appareillage est fondé sur un procédé très conventionnel de fabrication.

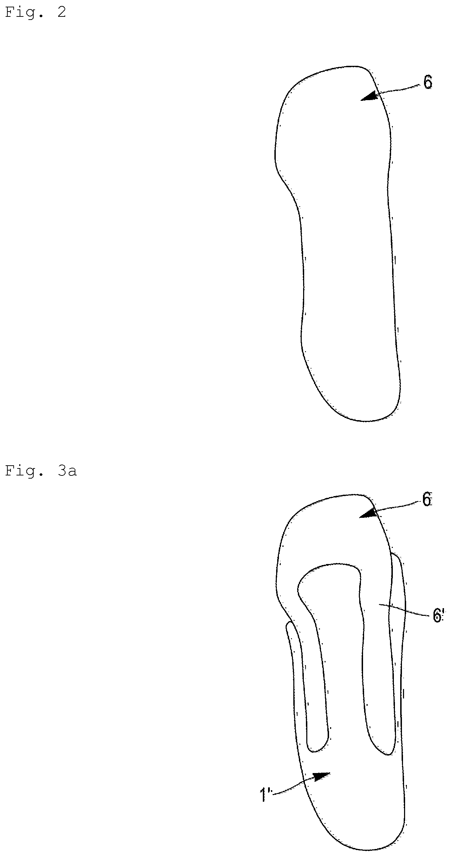

Ainsi, une première étape du procédé de fabrication de la prothèse prise en exemple comprend la fourniture, par n'importe quel moyen connu en soi, d'un positif 6 de la partie du corps d'un patient destinée à recevoir l'emboîture 100 (figure 2). Il peut s'agir d'un positif en plâtre, obtenu par moulage de la partie du corps en question.

La deuxième étape du procédé comprend la formation de la structure rigide 1, cette structure étant munie d'un insert 12 pour recevoir le tissu de liaison 3. La structure rigide 1 est formée uniquement sur une portion du positif 6 pour donner à cette structure une forme voulue, ici une forme comportant une pluralité de mâts 11. Une portion du positif 6, désignée portion libre 6', demeure ainsi libre de toute structure rigide 1. Une partie de cette portion libre 6' accueillera par la suite la couche flexible 2.

Cette deuxième étape de formation de la structure rigide 1 comprend plusieurs sous-étapes, représentées sur les figures 3a à 3d, que l'on va maintenant détailler. Dans une première sous-étape (figure 3a) on forme, sur la portion choisie du positif 6, une première couche 1' en un matériau apte à être rigidifié. Il peut ainsi s'agir de déposer sur le positif des bandes ou des feuilles de fibre, par exemple en carbone, imprégnées de résine polymère thermodurcissable. Alternativement, il peut aussi s'agir de feuilles ou de bandes d'une fibre technique (de nature listée dans une partie antérieure de cette description) que l'on imprègne, après les avoir apposés sur le positif, d'une résine polymère thermodurcissable ou thermo formable. La première couche 1' présente un contour 10 sensiblement conforme à la forme choisie de la structure rigide 1. La formation de la première couche 1' peut comprendre le dépôt d'une pluralité de couches intermédiaires, par exemple sous la forme de feuilles ou de bandes. La première couche 1' peut être ainsi composée d'un empilement de trois à dix couches intermédiaires, par exemple cinq couches intermédiaires, par exemple en fibre de carbone pré imprégnée de résine.

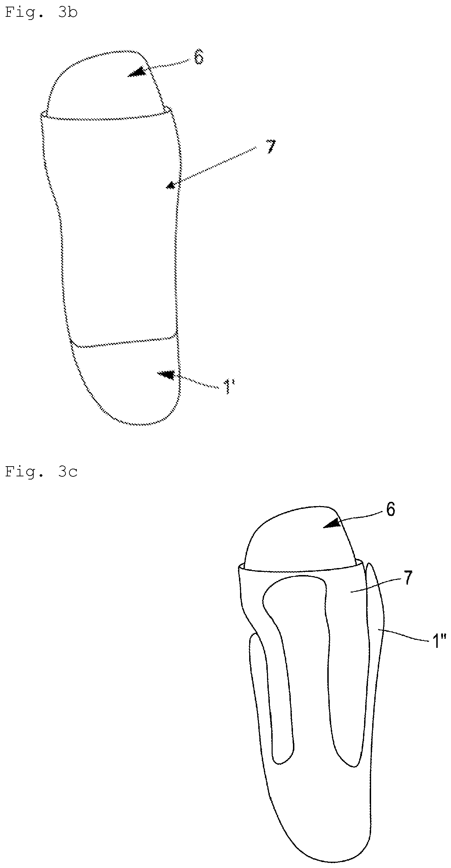

Dans une deuxième sous-étape (figure 3b) on dépose un film de séparation 7, par exemple en Téflon, sur au moins une partie de la première couche 1'. Le film de séparation 7 a pour but de délimiter les dimensions de l'insert 12 que l'on veut former dans la structure rigide 1 et qui permettra d'accueillir le tissu de liaison. Dans l'exemple représenté, le film de séparation recouvre toute la surface des mâts 11 sans recouvrir entièrement la base 11a. De manière plus générale, le film de séparation 7 peut avantageusement, afin de faciliter sa manipulation, dépasser de la première couche 1' et recouvrir la portion libre 6' du positif 6. Il est également envisageable que le film de séparation 7 recouvre entièrement la première couche 1' ou qu'il recouvre seulement une zone périphérique de cette première couche 1' s'étendant le long de son contour 10', selon les caractéristiques géométriques voulues pour l'insert 12.

Dans une troisième sous-étape (figure 3c) on forme une deuxième couche 1" en un matériau apte à être rigidifié, de la même manière que l'on a formé la première couche 1', en recouvrement de la première couche 1', de sorte que le film de séparation 7 soit disposé entre la première et la deuxième couche 1'. Dit autrement, le film de séparation 7 est pris en sandwich entre la première et la deuxième couche 1',1", ici dans la région destinée à former les mâts 11. La deuxième couche 1" peut également être formée de fibre de carbone imprégnée de résine thermodurcissable. La deuxième couche 1" peut être formée par un empilement de trois à dix couches intermédiaires, par exemple de cinq couches intermédiaires en fibre de carbone imprégnée de résine.

Dans une quatrième sous-étape, optionnelle, on applique un traitement thermique à l'ensemble formé de la première couche 1', du film de séparation 7 et de la deuxième couche 1" pour accélérer la réticulation de la résine et obtenir la structure rigide 1. Avantageusement, le traitement thermique est précédé d'une mise sous vide de cet ensemble. La mise sous vide permet de réaliser une bonne imprégnation des fibres par la résine composant le matériau de la première et de la deuxième couche 1',1" et permet de retirer la résine en excès afin de créer un matériau composite exempt d'imperfection. Le traitement thermique permet d'accélérer la polymérisation/le thermodurcissement de la résine. Celui-ci peut consister à porter l'ensemble à une température d'environ 100°C pendant une durée d'au moins une heure.

La présence du film de séparation 7 permet de prévenir le scellement de la première couche 1' à la deuxième couche 1" sur la portion occupée par ce film 7. On définit de la sorte l'insert 12 permettant de recevoir le tissu de liaison 3. Ainsi, dans l'exemple représenté, la première couche 1' et la deuxième couche 1" sont uniquement scellées entre elles au niveau de leur base 11a, mais pas au niveau des mâts 11. Il est à noter qu'il est envisageable que la première couche 1' et la deuxième couche 1" soient entièrement séparées l'une de l'autre dans le cas où le film de séparation 7 recouvre entièrement la première couche 1'. Dans une telle configuration, la structure rigide 1 est constituée de deux parties s'emboîtant parfaitement l'une dans l'autre.

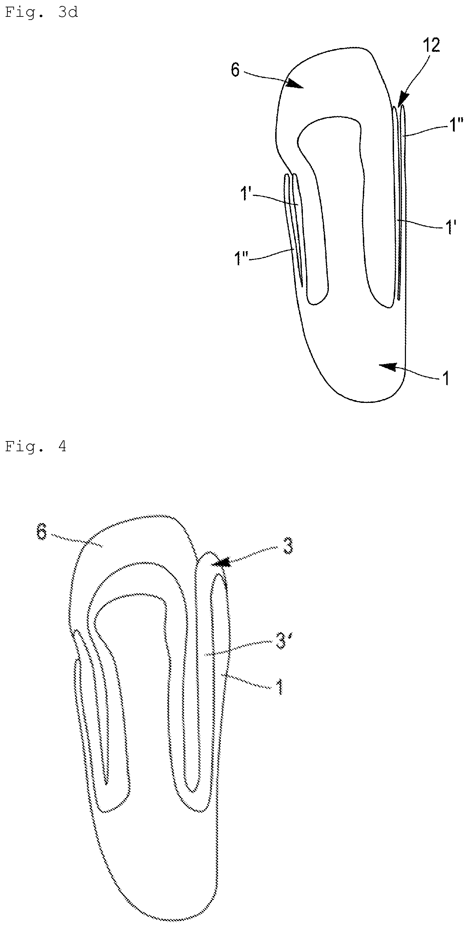

Dans une cinquième sous-étape (figure 3d), on extrait le film de séparation 7 de la structure rigide 1. On définit de la sorte un insert 12 dans la structure rigide 1, cet insert pouvant être exploité, comme cela sera exposé ultérieurement, pour venir y placer une portion d'un tissu de liaison 3.

L'étape de formation de la structure rigide 1 peut également comprendre une sous-étape supplémentaire de finition de la structure rigide 1. On retire dans ce cas la structure rigide 1 du positif 6, et celle-ci est détourée mécaniquement pour lui donner la forme souhaitée. Les bords de la structure rigide 1 peuvent être poncés et les surfaces externe et/ou interne peuvent également être lissées ou traitées pour en améliorer le confort et/ou la forme et favoriser son adhérence aux films de revêtement externes et internes quand ils sont présents. La structure rigide 1 finie est ensuite replacée sur le positif 6.

Au cours de la formation de la structure rigide 1, si cela est nécessaire, on peut assembler l'élément modulaire 4 et la valve 5 sur la structure rigide 1 selon des techniques connues de la personne du métier.

Poursuivant la description générale du procédé de fabrication de l'appareillage médical 100, celui-ci comprend une troisième étape de formation de la couche déformable 2. Cette étape comprend plusieurs sous-étapes que l'on va maintenant détailler.

Dans une première sous-étape de cette troisième étape, on engage une portion du tissu de liaison 3, par exemple en fibre de verre comme cela a déjà été évoqué, dans l'insert 12 de la structure rigide 1 (figure 4). Pour faciliter cette insertion, on peut écarter l'une de l'autre les deux parties rigides définissant l'insert 12. La partie restant du tissu de liaison 3, c'est-à-dire la partie non engagée dans l'insert 12, forme une portion exposée 3' qui recouvre une partie au moins de la portion libre 6' du positif 6. De manière avantageuse, le tissu de liaison 3 est préalablement enduit de colle sur la portion destinée à s'engager dans l'insert 12 afin de le solidariser à la structure rigide.

Dans une deuxième sous-étape, on dépose le matériau destiné à former la couche déformable 2, par exemple une gomme ou un gel de silicone comme cela a déjà été évoqué, sur le positif 6. Plus précisément, le matériau destiné à former la couche déformable 2 est disposé directement sur la portion exposée 3' du tissu de liaison 3 et, le cas échéant, directement sur la portion libre 6' du positif 6. La couche déformable 2 recouvre au moins en partie la portion exposée 3' du tissu de liaison 3 pour combler en matériau souple l'espace complémentaire à la structure rigide. La structure rigide 1 et la couche déformable 2 sont alors jointives, bord à bord. La couche déformable 2 complète la structure rigide 1 pour former l'appareillage médical 100.

Dans un mode de réalisation alternatif, on peut intervertir les deux sous étapes qui viennent d'être présentées. Dans ce cas, la couche déformable 2 est disposée directement sur une partie au moins de la portion libre 6' du positif 6, jointive à la structure rigide, avant d'insérer le tissu de liaison 3 dans l'insert 12. On fait en sorte que le matériau formant la couche rigide imprègne bien le tissu liaison 3.

Dans tous les cas, la couche déformable 2 complète la structure rigide 1 pour former l'appareillage médical 100, la structure rigide 1 et la couche déformable 2 étant disposées jointivement l'un contre l'autre, bord à bord.

On peut envisager de traiter la surface externe de la couche déformable 2 par exemple pour la rendre lisse et/ou lui donner un aspect particulier.

Dans une troisième sous-étape de la troisième étape de formation de la couche déformable 2, on applique un traitement thermique pour durcir la couche déformable 2. Avantageusement, le traitement thermique est précédé d'une mise sous vide qui permet de bien imprégner le tissu de liaison 3 avec le matériau de la couche déformable, d'assurer un bon contact entre cette couche 2 et le positif 6, ainsi qu'avec le bord de la structure rigide 1. Le traitement thermique peut correspondre à un étuvage, et permet de durcir la couche déformable 2. Il peut consister, selon la nature du matériau formant la couche déformable 2, à placer la couche déformable à une température d'environ 100°C pendant plusieurs minutes à plusieurs heures.

À l'issue de cette sous-étape, la couche déformable 2 est maintenue assemblée bord à bord avec la structure rigide 1 par le biais du tissu de liaison 3.

On dispose ainsi d'un appareillage flexible 100 complet, la couche déformable 2 étant juxtaposée à la structure rigide 1 au juste nécessaire pour compléter cette structure, sans matière à l'excès qui pourrait alourdir l'appareillage.

On peut bien entendu prévoir d'autres étapes de fabrication en complément de celles qui viennent d'être présentées. Ainsi, et selon une première variante, le procédé de fabrication peut comprendre une étape préliminaire, avant la formation de la structure rigide 1, de formation d'un film de revêtement interne. À cet effet, on réalise un film en un matériau déformable, par exemple une gomme de silicone, sur le positif 6. Puis, on applique un traitement thermique, préférentiellement un étuvage précédé d'une mise sous vide, pour durcir le film de revêtement interne. On forme ensuite un film d'isolation, par exemple en polychlorure de vinyle (PVC) ou en alcool polyvinylique (PVA), sur le film de revêtement interne pour l'isoler lors de la formation de la structure rigide 1 et empêcher leur adhésion. La présence du film d'isolation permet de facilement séparer la structure rigide 1 du film de revêtement interne pour permettre l'application de l'étape de finition à cette structure. Le film d'isolation est retiré à l'issue de la finition de la structure rigide 1, afin de mettre en contact la structure rigide avec le film de revêtement interne, lorsque cette structure rigide 1 est replacée sur le positif 6. On peut prévoir de coller l'un à l'autre la structure rigide 1 et le film de revêtement interne.

Selon une autre variante, le procédé de fabrication comprend une étape de formation d'un film de revêtement externe, après ou pendant la troisième étape de formation de la couche déformable 2. Cette étape comprend la formation d'un film en un matériau déformable, par exemple en gomme de silicone, sur la surface externe de la structure rigide 1. Cette étape est avantageusement réalisée pendant ou après la formation de la couche déformable 2 et avant son traitement thermique, celui-ci conduisant à durcir simultanément la couche déformable 2 et le film de revêtement externe.

Bien entendu l'invention n'est pas limitée au mode de mise en œuvre ainsi qu'aux variantes décrites et on peut y apporter d'autres variantes de réalisation sans sortir du cadre de l'invention tel que défini par les revendications.

L'invention n'est en particulier nullement limitée à la prothèse donnée en exemple et on peut envisager de concevoir sur les mêmes principes toute forme d'appareillage médical, prothèse ou orthèse, par exemple un corset ou une orthèse de main.

Il est bien entendu possible de donner une forme quelconque à la structure rigide, selon le besoin du patient et la nature de l'appareillage médical, et l'invention n'est nullement limitée à la structure rigide présentée sur les figures.

On peut également prévoir d'intégrer dans l'appareillage d'autres éléments que l'élément modulaire 4 et la valve 5, par exemple un élément permettant l'identification de cet appareillage, tel qu'une étiquette RFID.

D'autres manières de fabriquer cet appareillage peuvent bien évidemment être envisagées. On pourrait notamment envisager un procédé dans lequel les différents composants de l'appareillage sont empilés successivement sur le positif puis exposés à un traitement thermique. Par exemple, après avoir déposé la première couche en un matériau apte à être rigidifié sur le positif, la portion libre du positif est directement recouverte par une partie du matériau destiné à former la couche déformable. Ensuite le tissu de liaison est déposé sur la première couche de manière à avoir une portion « exposée » en contact avec le matériau destiné à former la couche déformable. Puis la deuxième couche en un matériau apte à être rigidifié est déposée sur la première couche pour prendre en sandwich le tissu de liaison. Pour finir, le reste du matériau destiné à former la couche déformable est déposé sur la partie précédemment déposée, et au moins partiellement recouverte par le tissu de liaison, pour former la couche déformable puis un traitement thermique est appliqué à l'ensemble pour finaliser l'appareillage.

## Revendications

1. Appareillage médical (100) flexible destiné à être disposé sur une partie du corps d'un patient et en épouser la forme, l'appareillage médical (100) flexible comprenant une structure rigide (1) présentant un contour (10) ainsi qu'au moins une couche déformable (2) et étant **caractérisé en ce qu'**il comprend un tissu de liaison (3) engagé dans au moins une portion périphérique de la structure rigide (1) s'étendant le long de son contour (10), le reste du tissu de liaison (3) formant une portion exposée (3') pour recevoir la couche déformable (2), la structure rigide (1) et la couche déformable (2) étant assemblées bord à bord, la couche déformable (2) complétant la structure rigide (1) pour former l'appareillage médical (100).

2. Appareillage médical (100) selon la revendication précédente dans lequel le matériau de la structure rigide (2) est un matériau composite à base de carbone.

3. Appareillage médical (100) selon l'une des revendications précédentes dans lequel le matériau du tissu de liaison (3) est de la fibre de verre.

4. Appareillage médical (100) selon l'une des revendications précédentes dans lequel le matériau de la couche déformable (2) est un matériau élastomère tel qu'un silicone.

5. Appareillage médical (100) selon l'une des revendications précédentes comprenant un film de revêtement interne en matériau déformable, le film de revêtement interne recouvrant au moins en partie la surface interne de la structure rigide (1) et la couche déformable (2).

6. Appareillage médical (100) selon l'une des revendications précédentes comprenant un film de revêtement externe en matériau déformable, le film de revêtement externe recouvrant au moins en partie la surface externe de la structure rigide (1) et la couche déformable (2).

7. Appareillage médical (100) selon l'une des revendications précédentes dans lequel la structure rigide (1) comprend au moins deux mâts (11) s'étendant sensiblement axialement depuis une base (11a) vers une extrémité (11b).

8. Procédé de fabrication d'un appareillage médical flexible (100) destiné à être disposé sur une partie du corps d'un patient et en épouser la forme, le procédé comprenant les étapes suivantes :
- fournir un positif (6) de la partie du corps destiné à recevoir l'appareillage médical (100) ;
- former une structure rigide (1) munie d'un insert (12) sur une portion seulement du positif (6) pour préserver une portion du positif (6), dite portion libre (6') ;
- former une couche déformable (2) sur la portion libre (6') du positif, **caractérisé en ce que** la formation de la couche déformable (2) comprend les étapes suivantes :
* engager une portion d'un tissu de liaison (3) dans l'insert (12) de la structure rigide (1), le reste du tissu de liaison (3) formant une portion exposée (3') recouvrant au moins partiellement la portion libre (6') du positif ;
* former la couche déformable (2) sur la portion exposée (3') du tissu de liaison (3) et bord à bord de la structure rigide (1) ;
* appliquer un traitement thermique pour durcir la couche déformable (2) .

9. Procédé de fabrication d'un appareillage médical (100) selon la revendication précédente dans lequel la formation de la structure rigide (2) comprend les étapes suivantes :
- former une première couche (1') en un matériau apte à être rigidifié, la première couche (1') présentant un contour (10);
- déposer un film de séparation (7) sur au moins une partie de la première couche (1') pour recouvrir au moins une zone périphérique s'étendant le long du contour (10) de cette première couche (1') ;
- former une deuxième couche (1") en un matériau apte à être rigidifié en recouvrement de la première couche (1'), de sorte que le film de séparation (7) soit disposé entre la première (1') et la deuxième couche (1") au niveau au moins de la zone périphérique ;
- appliquer un traitement thermique à l'ensemble formé de la première couche (1'), du film de séparation (7) et de la deuxième couche (1") pour obtenir la structure rigide (2), le film de séparation (7) prévenant le scellement de la première couche (1') à la deuxième couche (1") au moins dans la zone périphérique pour définir un insert (12) de la structure rigide (1) ;
- extraire le film de séparation (7) de la structure rigide (1) .

10. Procédé de fabrication d'un appareillage médical (100) selon la revendication précédente dans lequel la formation de la première couche (1') et de la deuxième couche (1") comprend le dépôt d'une pluralité de couches intermédiaires en matériau apte à être rigidifié.

11. Procédé de fabrication d'un appareillage médical (100) selon l'une des revendications 8 à 10 dans lequel la formation de la structure rigide (1) comprend, une étape de finition, l'étape de finition comprenant :
- le retrait du positif (6) ;
- le détourage de la structure rigide (1) pour conformer le contour (10) de la deuxième couche (1") à celui de la première couche (1') ;
- le ponçage des bords de la structure rigide (1) afin que les bords présentent une surface compatible avec un collage bord à bord ;
- la remise en place du positif (6).

12. Procédé de fabrication d'un appareillage médical (100) selon l'une des revendications 8 à 11 comprenant une étape préliminaire, avant la formation de la structure rigide (1), de formation d'un film de revêtement interne en matériau déformable, cette étape préliminaire comprenant :
- le dépôt du film de revêtement interne sur toute la surface du positif (6) ;
- un traitement thermique pour durcir le film de revêtement interne.

13. Procédé de fabrication d'un appareillage médical (100) selon la revendication précédente dans lequel l'étape préliminaire comprend, à la suite de son traitement thermique, le dépôt d'un film d'isolation sur le film de revêtement interne pour l'isoler du reste de l'appareillage médical (100) lors de la formation de la structure rigide, le film d'isolation étant retiré à l'issue de la formation de la structure rigide (1).

14. Procédé de fabrication d'un appareillage médical (100) selon l'une des revendications 8 à 11 comprenant une étape supplémentaire de dépôt d'un film de revêtement externe en matériau déformable pour recouvrir la surface externe de la structure rigide (1) ainsi que celle de la couche déformable (2) .

15. Procédé de fabrication d'un appareillage médical (100) selon la revendication précédente dans lequel l'étape supplémentaire de formation d'un film de revêtement externe est réalisée entre le dépôt de la couche déformable (2) et le traitement thermique de l'étape de formation de la couche déformable (2), le traitement thermique de l'étape de formation de la couche déformable durcissant simultanément la couche déformable (2) et le film de revêtement externe.

## Patentansprüche

1. Flexible medizinische Vorrichtung (100), die dazu bestimmt ist, auf einem Teil des Körpers eines Patienten angeordnet zu werden und sich an dessen Form anzupassen, die flexible medizinische Vorrichtung (100) umfassend eine steife Struktur (1), die eine Kontur (10) aufweist, sowie mindestens eine verformbare Schicht (2) und **dadurch gekennzeichnet, dass** sie ein Verbindungsgewebe (3) umfasst, das mit mindestens einem Umfangsabschnitt der steifen Struktur (1) in Eingriff steht, der sich entlang ihrer Kontur (10) erstreckt, wobei der Rest des Verbindungsgewebes (3) einen freiliegenden Abschnitt (3') zum Aufnehmen der verformbaren Schicht (2) ausbildet, wobei die steife Struktur (1) und die verformbare Schicht (2) Kante an Kante zusammengefügt sind, wobei die verformbare Schicht (2) die steife Struktur (1) zum Ausbilden der medizinischen Vorrichtung (100) ergänzt.

2. Medizinische Vorrichtung (100) nach dem vorstehenden Anspruch, wobei das Material der steifen Struktur (2) ein Verbundwerkstoff auf Kohlenstoffbasis ist.

3. Medizinische Vorrichtung (100) nach einem der vorstehenden Ansprüche, wobei das Material des Verbindungsgewebes (3) Glasfaser ist.

4. Medizinische Vorrichtung (100) nach einem der vorstehenden Ansprüche, wobei das Material der verformbaren Schicht (2) ein Elastomermaterial wie zum Beispiel Silikon ist.

5. Medizinische Vorrichtung (100) nach einem der vorstehenden Ansprüche, umfassend einen Innenbeschichtungsfilm aus verformbarem Material, wobei der Innenbeschichtungsfilm mindestens teilweise die Innenoberfläche der steifen Struktur (1) und der verformbaren Schicht (2) bedeckt.

6. Medizinische Vorrichtung (100) nach einem der vorstehenden Ansprüche, umfassend einen Außenbeschichtungsfilm aus verformbarem Material, wobei der Außenbeschichtungsfilm mindestens teilweise die Außenoberfläche der steifen Struktur (1) und der verformbaren Schicht (2) bedeckt.

7. Medizinische Vorrichtung (100) nach einem der vorstehenden Ansprüche, wobei die steife Struktur (1) mindestens zwei Stangen (11) umfasst, die sich im Wesentlichen axial von einer Basis (11a) in Richtung eines Endes (11b) erstrecken.

8. Verfahren zum Herstellen einer flexiblen medizinischen Vorrichtung (100), die dazu bestimmt ist, auf einem Teil des Körpers eines Patienten angeordnet zu werden und sich an dessen Form anzupassen, das Verfahren umfassend die folgenden Schritte:
- Bereitstellen eines Positivs (6) des Teils des Körpers, der dazu bestimmt ist, die medizinische Vorrichtung (100) aufzunehmen;
- Ausbilden einer steifen Struktur (1), die mit einem Einsatz (12) auf einem Abschnitt nur des Positivs (6) zum Bewahren des Abschnitts des Positivs (6), dem sogenannten freien Abschnitt (6'), versehen ist;
- Ausbilden einer verformbaren Schicht (2) auf dem freien Abschnitt (6') des Positivs, **dadurch gekennzeichnet, dass** das Ausbilden der verformbaren Schicht (2) die folgenden Schritte umfasst:
* Ineingriffnehmen eines Abschnitts des Verbindungsgewebes (3) in dem Einsatz (12) der steifen Struktur (1), wobei der Rest des Verbindungsgewebes (3) einen freiliegenden Abschnitt (3') ausbildet, der mindestens teilweise den freien Abschnitt (6') des Positivs bedeckt; * Ausbilden der verformbaren Schicht (2) auf dem freiliegenden Abschnitt (3') des Verbindungsgewebes (3) und Kante an Kante der steifen Struktur (1);
* Anwenden einer Wärmebehandlung zum Aushärten der verformbaren Schicht (2).

9. Verfahren zum Herstellen einer medizinischen Vorrichtung (100) nach dem vorstehenden Anspruch, wobei das Ausbilden der steifen Struktur (2) die folgenden Schritte umfasst:
- Ausbilden einer ersten Schicht (1') aus einem Material, das geeignet ist, um versteift zu werden, wobei die erste Schicht (1') eine Kontur (10) aufweist;
- Abscheiden eines Trennfilms (7) auf mindestens einen Teil der ersten Schicht (1') zum Bedecken mindestens einer Umfangszone, die sich entlang der Kontur (10) dieser ersten Schicht (1') erstreckt;
- Ausbilden einer zweiten Schicht (1") aus einem Material, das geeignet ist, um in Bedeckung der ersten Schicht (1') so versteift zu werden, dass der Trennfilm (7) zwischen der ersten (1') und der zweiten Schicht (1") an mindestens der Umfangszone angeordnet ist;
- Anwenden einer Wärmebehandlung auf die Einheit, die aus der ersten Schicht (1'), dem Trennfilm (7) und der zweiten Schicht (1") ausgebildet wird, zum Erhalten der steifen Struktur (2), wobei der Trennfilm (7) die Versiegelung der ersten Schicht (1') mit der zweiten Schicht (1") mindestens in der Umfangszone zum Definieren eines Einsatzes (12) der steifen Struktur (1) verhindert;
- Extrahieren der Trennfolie (7) von der steifen Struktur (1).

10. Verfahren zum Herstellen einer medizinischen Vorrichtung (100) nach dem vorstehenden Anspruch, wobei das Ausbilden der ersten Schicht (1') und der zweiten Schicht (1") das Abscheiden einer Vielzahl von Zwischenschichten aus einem Material umfasst, das geeignet ist, um versteift zu werden.

11. Verfahren zum Herstellen einer medizinischen Vorrichtung (100) nach einem der Ansprüche 8 bis 10, wobei das Ausbilden der steifen Struktur (1) einen Fertigstellungsschritt umfasst, der Fertigstellungsschritt umfassend:
- Entfernen des Positivs (6);
- Beschneiden der steifen Struktur (1) zum Inübereinstimmungbringen der Kontur (10) der zweiten Schicht (1") mit der der ersten Schicht (1');
- Schleifen der Kanten der steifen Struktur (1), damit die Kanten eine Oberfläche aufweisen, die mit einem Kleben Kante an Kante kompatibel sind;
- Wiederaufsetzen des Positivs (6).

12. Verfahren zum Herstellen einer medizinischen Vorrichtung (100) nach einem der Vorsprüche 8 bis 11, umfassend einen Vorbereitungsschritt, vor dem Ausbilden der steifen Struktur (1), des Ausbildens eines Innenbeschichtungsfilms aus verformbarem Material, dieser Vorbereitungsschritt umfassend:
- Abscheiden des Innenbeschichtungsfilms auf die gesamte Oberfläche des Positivs (6);
Wärmebehandeln zum Härten des Innenbeschichtungsfilms.

13. Verfahren zum Herstellen einer medizinischen Vorrichtung (100) nach dem vorstehenden Anspruch, wobei der Vorbereitungsschritt nach seiner Wärmebehandlung das Abscheiden eines Isolationsfilms auf den Innenbeschichtungsfilm zum Isolieren des Rests der medizinischen Vorrichtung (100) bei dem Ausbilden der steifen Struktur umfasst, wobei der Isolierfilm am Ende des Ausbildens der steifen Struktur (1) entfernt wird.

14. Verfahren zum Herstellen einer medizinischen Vorrichtung (100) nach einem der Ansprüche 8 bis 11, umfassend einen zusätzlichen Schritt des Abscheidens eines Außenbeschichtungsfilms aus verformbarem Material zum Bedecken der Außenoberfläche der steifen Struktur (1) sowie derjenigen der verformbaren Schicht (2).

15. Verfahren zum Herstellen einer medizinischen Vorrichtung (100) nach dem vorstehenden Anspruch, wobei der zusätzliche Schritt des Ausbildens eines Außenbeschichtungsfilms zwischen dem Abscheiden der verformbaren Schicht (2) und dem Wärmebehandeln des Schritts des Ausbildens der verformbaren Schicht (2) erfolgt, wobei das Wärmebehandeln des Schritts des Ausbildens der verformbaren Schicht gleichzeitig die verformbare Schicht (2) und den Außenbeschichtungsfilm härtet.

## Claims

1. A flexible medical appliance (100) intended to be placed on a part of a patient's body and to match its shape, the flexible medical appliance (100) comprising a rigid structure (1) having a contour (10), as well as at least one deformable layer (2), and being **characterized in that** it comprises a bonding fabric (3) engaged in at least one peripheral portion of the rigid structure (1) extending along its contour (10), the rest of the bonding fabric (3) forming an exposed portion (3') to receive the deformable layer (2), the rigid structure (1) and the deformable layer (2) being assembled edge to edge, the deformable layer (2) completing the rigid structure (1) to form the medical appliance (100).

2. The medical appliance (100) according to the preceding claim, wherein the material of the rigid structure (2) is a carbon-based composite material.

3. The medical appliance (100) according to any of the preceding claims, wherein the material of the bonding fabric (3) is fiberglass.

4. The medical appliance (100) according to any of the preceding claims, wherein the material of the deformable layer (2) is an elastomer material such as a silicone.

5. The medical appliance (100) according to any of the preceding claims, comprising an inner coating film of deformable material, the inner coating film at least partially covering the inner surface of the rigid structure (1) and the deformable layer (2).

6. The medical appliance (100) according to any of the preceding claims, comprising an outer coating film of deformable material, the outer coating film at least partially covering the outer surface of the rigid structure (1) and the deformable layer (2).

7. The medical appliance (100) according to any of the preceding claims, wherein the rigid structure (1) comprises at least two posts (11) extending substantially axially from a base (11a) towards an end (11b).

8. A method for manufacturing a flexible medical appliance (100) intended to be placed on a part of a patient's body and to match its shape, the method comprising the following steps:
- providing a positive mold (6) of the part of the body intended to receive the medical appliance (100);
- forming a rigid structure (1), provided with an insert (12) on only one portion of the positive mold (6) to preserve a portion of the positive mold (6) called the free portion (6');
- forming a deformable layer (2) on the free portion (6') of the positive mold, **characterized in that** the formation of the deformable layer (2) comprises the following steps:
* engaging a portion of a bonding fabric (3) in the insert (12) of the rigid structure (1), the rest of the bonding fabric (3) forming an exposed portion (3') at least partially covering the free portion (6') of the positive mold; * forming the deformable layer (2) on the exposed portion (3') of the bonding fabric (3) and edge to edge of the rigid structure (1);
* applying a heat treatment to harden the deformable layer (2).

9. The method for manufacturing a medical appliance (100) according to the preceding claim, wherein the formation of the rigid structure (2) comprises the following steps:
- forming a first layer (1') of a material able to be stiffened, the first layer (1') having a contour (10);
- arranging a separation film (7) on at least a part of the first layer (1') to cover at least one peripheral zone extending along the contour (10) of this first layer (1');
- forming a second layer (1") of a material capable of being stiffened overlapping the first layer (1') so that the separation film (7) is placed between the first layer (1') and the second layer (1") at least at the peripheral zone;
- applying a heat treatment to the assembly formed by the first layer (1'), the separation film (7) and the second layer (1") to obtain the rigid structure (2), the separation film (7) preventing the sealing of the first layer (1') to the second layer (1") at least in the peripheral zone to define an insert (12) in the rigid structure (1);
- extracting the separation film (7) from the rigid structure (1).

10. The method for manufacturing a medical appliance (100) according to the preceding claim, wherein the formation of the first layer (1') and of the second layer (1") comprises the arrangement of a plurality of intermediate layers of material capable of being stiffened.

11. The method for manufacturing a medical appliance (100) according to any of claims 8 to 10, wherein the formation of the rigid structure (1) comprises a finishing step, the finishing step comprising:
- removing the positive mold (6);
- trimming the rigid structure (1) to shape the contour (10) of the second layer (1") to that of the first layer (1');
- sanding the edges of the rigid structure (1) so that the edges have a surface compatible with edge-to-edge bonding;
- putting the positive mold (6) back in place.

12. The method for manufacturing a medical appliance (100) according to any of claims 8 to 11, comprising a preliminary step, before forming the rigid structure (1), of forming an inner coating film of deformable material, this preliminary step comprising:
- arranging the inner coating film over the entire surface of the positive mold (6);
- a heat treatment to harden the inner coating film.

13. The method for manufacturing a medical appliance (100) according to the preceding claim, wherein the preliminary step comprises, following its heat treatment, the arrangement of an isolating film on the inner coating film to isolate it from the rest of the medical appliance (100) during the formation of the rigid structure, the isolating film being removed after the formation of the rigid structure (1).

14. The method for manufacturing a medical appliance (100) according to any of claims 8 to 11, comprising an additional step of arranging an outer coating film of deformable material to cover the outer surface of the rigid structure (1) as well as that of the deformable layer (2).

15. The method for manufacturing a medical appliance (100) according to the preceding claim, wherein the additional step of forming an outer coating film is carried out between the arrangement of the deformable layer (2) and the heat treatment of the step of forming the deformable layer (2), the heat treatment of the step of forming the deformable layer simultaneously curing the deformable layer (2) and the outer coating film.
